# EUROPEAN PATENT APPLICATION

(11) **EP 4 474 010 A1**
(43) Date of publication of application: **11.12.2024**
(21) Application number: 24180917.7
(22) Date of filing: 07.06.2024
(51) Int. Cl.: A61N 5/10

(54) **METHOD FOR USE WITH A RADIOTHERAPY DEVICE**

(30) Priority: 07.06.2023 GB 202308500
(71) Applicant: Elekta Limited, Crawley, Sussex RH10 9RR (GB)
(72) Inventor: Flint, Christopher, Crawley, RH10 9RR (GB); Lingxia, Chen, Crawley, RH10 9RR (GB)
(74) Representative: Hamer, Thomas Daniel

(57) **Abstract**

Disclosed herein is a method of determining whether repair or replacement of thyratron of a radiotherapy device should be scheduled. The radiotherapy device comprising a linear accelerator and being configured to provide therapeutic radiation to a patient. The radiotherapy device comprising a thyratron; and at least one sensor configured to provide a signal indicative of a start-up time associated with the thyratron. The method comprises receiving the start-up time and based on the start-up time, determining whether repair or replacement of the thyratron should be scheduled.

## Description

This disclosure relates to the fields of predictive maintenance and remote diagnostics, and in particular to methods of determining whether to schedule repair or replacement of thyratron in a radiotherapy device, and determining whether maintenance of a radiotherapy device comprising the thyratron should be scheduled.

### Background

Radiotherapy devices are an important tool in modern cancer treatment. Radiotherapy devices are large, complex machines, with many moving parts and inter-operating mechanisms. Despite precision engineering and rigorous testing, some component parts of a radiotherapy device may start to degrade over its lifetime. This can sometimes lead to sub-optimal operation and even the occasional safety override.

If at any point during treatment a radiotherapy device starts to function outside of its normal operating parameters, a safety override or "interrupt" occurs, whereby the machine stops delivering radiation to ensure patient safety. Such an event is inconvenient, as it adds time to the treatment, and in some cases means the treatment session must finish prematurely. Unplanned equipment downtime can disrupt planned treatment schedules, and may be expensive for the owner, be it due to loss of revenue, servicing and repair costs, or both.

It has been surmised that predictive maintenance and/or remote diagnostic techniques could be applied to radiotherapy machines. However, given the complexity of the machines and the sheer volume of data which may be gathered during operation, it is difficult to know how to analyse any available data to inform the predictive maintenance techniques. Even when a problematic machine is identified, trying to ascertain the nature of the fault is very difficult given the abundance of data and the complex interrelationships between the various components of the machine. In other words, even if a wealth of data from a radiotherapy device is available, remotely determining the nature of a fault or assessing the condition of a particular component is not a trivial matter.

The present disclosure relates, in part, to identifying that a thyratron of a radiotherapy device is nearing the time at which it should be replaced or repaired. To date, no such predictive approach has been possible, and existing methods of servicing and repair involve noting that a particular device has undergone multiple safety overrides, and/or waiting until a thyratron is faulty and operating sub-optimally such that a radiotherapy machine cannot safely begin operation, and sending a field service engineer to inspect the machine and diagnose and fix the problem. Often, the type of problem or the component which is at fault is not known in advance, and hence time consuming diagnostic testing must be performed on-site. Existing methods therefore result in a significant amount of machine down time. Also, in existing methods, a field service engineer is not made aware of a potential issue until the component has degraded to a point where the radiotherapy machine is undergoing safety interrupts, or even until the point where the radiotherapy machine cannot operate within its safety parameters. This means that the servicing of the radiotherapy machine is often scheduled at a time which is inconvenient, or inefficient in terms of both field service engineer resources and the resources of the hospital or other machine owner.

The present invention seeks to address these and other disadvantages encountered in the prior art by providing methods of determining, preferably remotely, whether repair or replacement of a thyratron in a radiotherapy device should be scheduled, determining the nature of a fault in a radiotherapy device and determining whether maintenance of a radiotherapy device should be scheduled.

### Summary

An invention is set out in the independent claims. Optional features are set out in the dependent claims.

According to an aspect of the invention there is provided a method of determining whether repair or replacement of thyratron of a radiotherapy device should be scheduled. The radiotherapy device comprises a linear accelerator and is configured to provide therapeutic radiation to a patient. The radiotherapy device comprises a thyratron and at least one sensor configured to provide a signal indicative of a start-up time associated with the thyratron. The method comprises receiving the start-up time and, based on the start-up time, determining whether repair or replacement of the thyratron should be scheduled.

The method may further comprise processing the start-up time and based on the processing, determining whether repair or replacement of the thyratron should be scheduled.

Processing the start-up time may comprise determining whether the start-up time meets at least one threshold time criterion.

Processing the start-up time may comprise determining whether a rate of change of the start-up time meets at least one threshold rate of change criterion.

Processing the start-up time may comprise determining whether the start-up time meets a first threshold criterion; and determining whether the rate of change of the start-up time meets a first threshold rate of change criterion; if the first threshold time criterion and first threshold rate of change criterion are met, determining that repair or replacement of the thyratron should be scheduled.

Processing the start-up time may comprise determining that the first threshold rate of change criterion is not met, and determining whether the start-up time meets a second threshold time criterion, wherein the second threshold time criterion is greater than the first threshold time criterion. It may comprise determining whether the rate of change of the start-up time meets a second threshold rate of change criterion, wherein the second threshold rate of change criterion is less than the first threshold rate of change criterion. If the second threshold time criterion and the second threshold rate of change criterion are met, determining that repair or replacement of the thyratron should be scheduled.

The thyratron may comprise an anode, a cathode heater, wherein a cathode heater voltage is associated with the cathode heater, and a first grid positioned between the cathode and the anode, wherein a first voltage is associated with the first grid.

The thyratron may comprise a second grid positioned between the cathode and the anode, wherein a second voltage is associated with the second grid.

The thyratron may comprise a replenisher, wherein a replenisher voltage is associated with the replenisher.

The signal indicative of the start-up time may be based on the value of at least one of the first voltage, the cathode heater voltage, the second voltage, and / or the replenisher voltage.

The signals indicative of the start-up time may be based on each of the first voltage, the second voltage, the replenisher voltage and the cathode heater voltage each being within a threshold range of values, each threshold range being defined by an upper range value and a lower range value.

The start-up time may be an average of signals received from the sensor over an averaging time period.

Determining whether the rate of change meets the threshold rate of change criterion may comprise calculating a linear coefficient of the rate of change of the start-up time over a rolling time period. The rolling time period may be greater than 24 hours, and optionally greater than three days, and optionally greater than seven days, and optionally greater than thirty days.

The method may further comprise outputting the determination of whether repair or replacement of the thyratron should be scheduled.

Outputting the determination may comprise at least one of displaying an indication of the determination on a display screen, issuing an alert detailing the determination to a field service engineer, and/or automatically issuing a notification to the owner or operator of the radiotherapy device to schedule the repair or replacement.

According to a further aspect of the invention there is provided a computer-readable medium comprising computer-executable instructions which, when executed by a processor, cause the processor to perform the above methods.

According to a further aspect of the invention there is provided a system comprising a remote controller communicatively coupled to a central controller and a device controller via a network. The central controller is configured to receive data from a radiotherapy device. The radiotherapy device comprises a linear accelerator, and is configured to provide therapeutic radiation to a patient. It comprises a thyratron and a sensor configured to provide at least one signal indicative of start-up time of the thyratron. The central controller, the remote controller and/or a device controller are configured to perform the above method steps.

### Figures

Specific embodiments are now described, by way of example only, with reference to the drawings, in which:
Figure 1 depicts a schematic illustration of a radiotherapy device comprising a Linac;
Figure 2 depicts a system according to the present disclosure;
Figure 3 depicts a thyratron;
Figure 4 is a flowchart which depicts a method according to the present disclosure;
Figure 5 is a flowchart which depicts a further method according to the present disclosure;
Figure 6 depicts start-up time data for a given thyratron assigned label 1;
Figure 7 depicts start-up time data for a given thyratron assigned label 2;
Figure 8 depicts start-up time data for a given thyratron assigned label 3;
Figure 9 depicts start-up time data for a given thyratron assigned label 4;
Figure 10 is a block diagram of an implementation of a computing device.

### Detailed Description

The present disclosure relates to a method determining whether a component or components of a radiotherapy machine or device should be serviced or replaced, and/or to determining to what degree such a component is operating within safety parameters and optimal operational parameters. The radiotherapy device may be suitable for delivering a beam of radiation to a patient in order to treat a tumour. An example of a radiation source for producing a therapeutic beam of radiation is a linear accelerator (Linac). Clinical Linac devices are configured to deliver high energy radiation to a patient.

Radiotherapy machines are beginning to be configured to produce and record a large amount of data as they operate; for example, radiotherapy machines are configured to provide sensor readings from a variety of different sensors. These sensors produce data which can be stored in a database. Radiotherapy devices may also be configured to allow remote connection, enabling service engineers to access a wealth of information about any connected machine without having to travel to the site where the machine is located. It is expected that, in many cases, machines may be returned to optimal performance without an engineer ever having to physically interact with the machine. However, there will still be occasions where the fault cannot be fixed remotely, and an engineer must be sent to: inspect the machine; determine the nature of the fault; and perform any maintenance required. If the repair involves replacing a part, further machine downtime is required before the machine can be brought back online.

Some of the present methods involve evaluating the condition and/or performance of a component of radiotherapy equipment during its operation in order to identify and determine, preferably remotely, whether the component is nearing the end of its operational life and thus whether the component should be replaced or repaired. Others of the present methods involve determining the nature of a fault in a radiotherapy device or determining whether maintenance of a radiotherapy device should be scheduled. The present application relates, in part, to determining whether a thyratron is approaching the end of its operational life. Such techniques are advantageous as they allow a manufacturer or maintenance service provider to attend the machine knowing what will be required to fix the machine prior to arrival. Some of the disclosed techniques allow the operation of the thyratron to be monitored, and hence thyratrons which are approaching the end of their operational life but which are still operating within required safety parameters can be identified. This in turn allows, for example, repair and/or replacement of the thyratron to be scheduled for the next convenient service point. The disclosed methods help to reduce machine downtime and thereby minimise disruption to the machine's normal operation. The disclosed techniques can also be used to more effectively plan machine downtime for times which are more convenient or cost-effective for the owner of the equipment and/or the patients.

As outlined above, the present application relates, in part, to determining the nature of a fault in a radiotherapy device, in particular, to determine whether there is a fault with the thyratron that means it will have to be repaired or replaced imminently. Typically, this determination would be based on the number of high tension (HT) hours of a given thyratron. However, this can lead to thyratrons being replaced prematurely. The present application relates to, in part, determining whether there is a fault based on the start-up time of the thyratron. Such techniques are advantageous as they allow a manufacturer or maintenance service provider to attend the machine knowing what will be required to fix the machine prior to arrival. The disclosed techniques can help to reduce machine downtime and thereby minimise disruption to the machine's normal operation. The disclosed techniques can also be used to more effectively plan machine downtime for times which are more convenient or cost-effective for the owner of the equipment and/or the patients.

As outlined above, the present application relates, in part, to determining whether maintenance of a radiotherapy device should be scheduled, in particular, as a result of issues with the replenisher of the thyratron of the radiotherapy device. The operating lifetime of a thyratron can be negatively affected by a poor vacuum quality in the vacuum tube. As such, identifying that the thyratron is reaching the end of its operational lifetime and scheduling maintenance of a radiotherapy device to address the issues can extend the operational lifetime of the radiotherapy device. For example, re-tapping may be utilized to address these issues. Alternatively, a filament may need to be replaced. Alternatively, the entire thyratron may need to be replaced.

As is known to the skilled person, a Linac may comprise a thyratron to function as a high-voltage electrical switch or control element to control, and trigger, the beam of therapeutic beam of radiation. A thyratron discharges a pulse forming network through a pulse transformer. A powered on thyratron is one in which the therapeutic radiation beam is triggered. Conversely, a standby mode or powered off thyratron is one where there is no emission of high-energy electrons forming a radiation beam. The start-up time or warm-up time is the time taken for the thyratron to reach suitable operating conditions. In particular, this may be the time taken for certain voltages to be within acceptable operation ranges, measured from the turn-on-time of the thyratron. This is typically measured in seconds.

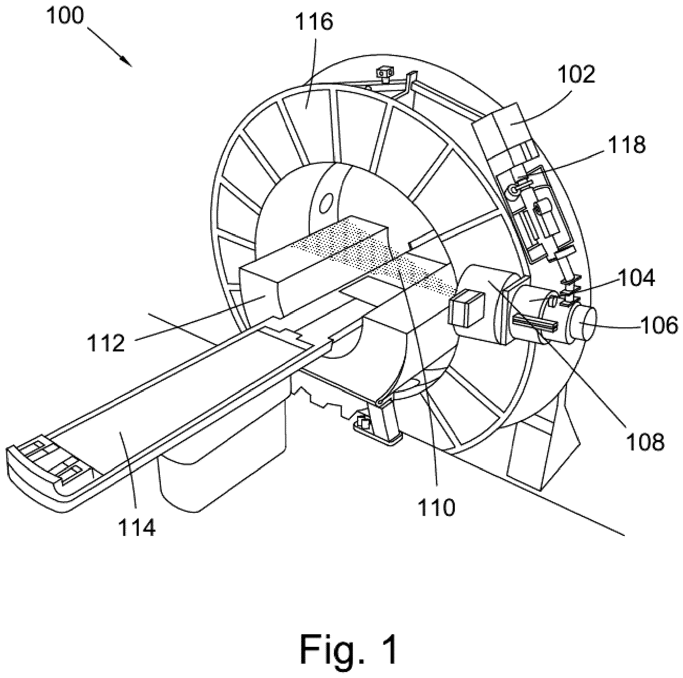

Figure 1 depicts a radiotherapy device suitable for delivering, and configured to deliver, a beam of radiation to a patient during radiotherapy treatment. The device and its constituent components will be described generally for the purpose of providing useful accompanying information for the present invention. The device depicted in figure 1 is in accordance with the present disclosure and is suitable for use with the disclosed systems and apparatuses. While the device in figure 1 is an MR-Linac, the implementations of the present disclosure may be any radiotherapy device, for example a Linac device.

The device 100 depicted in figure 1 is an MR-Linac. The device 100 comprises both MR imaging apparatus 112 and radiotherapy (RT) apparatus which may comprise a Linac device. The MR imaging apparatus 112 is shown in cross-section in the diagram. In operation, the MR scanner produces MR images of the patient, and the Linac device produces and shapes a beam of radiation and directs it toward a target region within a patient's body in accordance with a radiotherapy treatment plan. The depicted device does not have the usual 'housing' which would cover the MR imaging apparatus 112 and RT apparatus in a commercial setting such as a hospital. The Linac may further comprise a thyratron to function as a high-voltage electrical switch or control element to control, and trigger, the beam of therapeutic beam of radiation.

The MR-Linac device depicted in figure 1 comprises a source of radiofrequency waves 102, a waveguide 104, a source of electrons 106, a source of radiation 106, a collimator 108 such as a multi-leaf collimator configured to collimate and shape the beam, MR imaging apparatus 112, and a patient support surface 114. In use, the device would also comprise a housing (not shown) which, together with the ring-shaped gantry, defines a bore. The moveable support surface 114 can be used to move a patient, or other subject, into the bore when an MR scan and/or when radiotherapy is to commence. The MR imaging apparatus 112, RT apparatus, and a subject support surface actuator are communicatively coupled to a controller or processor. The controller is also communicatively coupled to a memory device comprising computer-executable instructions which may be executed by the controller.

The RT apparatus comprises a source of radiation and a radiation detector (not shown). Typically, the radiation detector is positioned diametrically opposed to the radiation source. The radiation detector is suitable for, and configured to, produce radiation intensity data. In particular, the radiation detector is positioned and configured to detect the intensity of radiation which has passed through the subject. The radiation detector may also be described as radiation detecting means, and may form part of a portal imaging system.

The radiation source may comprise a beam generation system. For a Linac, the beam generation system may comprise a source of RF energy 102, an electron gun 106, and a waveguide 104. The radiation source is attached to the rotatable gantry 116 so as to rotate with the gantry 116. In this way, the radiation source is rotatable around the patient so that the treatment beam 110 can be applied from different angles around the gantry 116. In a preferred implementation, the gantry is continuously rotatable. In other words, the gantry can be rotated by 360 degrees around the patient, and in fact can continue to be rotated past 360 degrees. The gantry may be ring-shaped. In other words, the gantry may be a ring-gantry.

The source 102 of radiofrequency waves, such as a magnetron, is configured to produce radiofrequency waves. The source 102 of radiofrequency waves is coupled to the waveguide 104 via circulator 118, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source and the waveguide 104 is such that the radiofrequency waves accelerate the electrons to very high energies as the electrons propagate through the waveguide 104.

The design of the waveguide 104 depends on whether the Linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

Once the electrons have been accelerated, they may pass into a flight tube. The flight tube may be connected to the waveguide by a connecting tube. This connecting tube or connecting structure may be called a drift tube. The electrons travel toward a heavy metal target which may comprise, for example, tungsten. Whilst the electrons travel through the flight tube, an arrangement of focusing magnets act to direct and focus the beam on the target.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun. Electrons can be accelerated to speeds approaching the speed of light in the evacuated waveguide 104.

The source of radiation is configured to direct a beam 110 of therapeutic radiation toward a patient positioned on the patient support surface 114. The source of radiation may comprise a heavy metal target toward which the high energy electrons exiting the waveguide are directed. When the electrons strike the target, X-rays are produced in a variety of directions. A primary collimator may block X-rays travelling in certain directions and pass only forward travelling X-rays to produce a treatment beam 110. The X-rays may be filtered and may pass through one or more ion chambers for dose measuring. The beam can be shaped in various ways by beam-shaping apparatus, for example by using a multi-leaf collimator 108, before it passes into the patient as part of radiotherapy treatment.

In some implementations, the source of radiation is configured to emit either an X-ray beam or an electron particle beam. Such implementations allow the device to provide electron beam therapy, i.e. a type of external beam therapy where electrons, rather than X-rays, are directed toward the target region. It is possible to `swap' between a first mode in which X-rays are emitted and a second mode in which electrons are emitted by adjusting the components of the Linac. In essence, it is possible to swap between the first and second mode by moving the heavy metal target in or out of the electron beam path and replacing it with a so-called 'electron window'. The electron window is substantially transparent to electrons and allows electrons to exit the flight tube.

The subject or patient support surface 114 is configured to move between a first position substantially outside the bore, and a second position substantially inside the bore. In the first position, a patient or subject can mount the patient support surface. The support surface 114, and patient, can then be moved inside the bore, to the second position, in order for the patient to be imaged by the MR imaging apparatus 112 and/or imaged or treated using the RT apparatus. The movement of the patient support surface is effected and controlled by a subject support surface actuator, which may be described as an actuation mechanism. The actuation mechanism is configured to move the subject support surface in a direction parallel to, and defined by, the central axis of the bore. The terms subject and patient are used interchangeably herein such that the subject support surface can also be described as a patient support surface. The subject support surface may also be referred to as a moveable or adjustable couch or table.

The radiotherapy apparatus / device depicted in figure 1 also comprises MR imaging apparatus 112. The MR imaging apparatus 112 is configured to obtain images of a subject positioned, i.e. located, on the subject support surface 114. The MR imaging apparatus 112 may also be referred to as the MR imager. The MR imaging apparatus 112 may be a conventional MR imaging apparatus operating in a known manner to obtain MR data, for example MR images. The skilled person will appreciate that such a MR imaging apparatus 112 may comprise a primary magnet, one or more gradient coils, one or more receive coils, and an RF pulse applicator. The operation of the MR imaging apparatus is controlled by the controller.

The controller is a computer, processor, or other processing apparatus. The controller may be formed by several discrete processors; for example, the controller may comprise an MR imaging apparatus processor, which controls the MR imaging apparatus 110; an RT apparatus processor, which controls the operation of the RT apparatus; and a subject support surface processor which controls the operation and actuation of the subject support surface. The controller is communicatively coupled to a memory, e.g. a computer readable medium.

The Linac device also comprises several other components and systems as will be understood by the skilled person. For example, in order to ensure the Linac does not leak radiation, appropriate shielding is also provided.

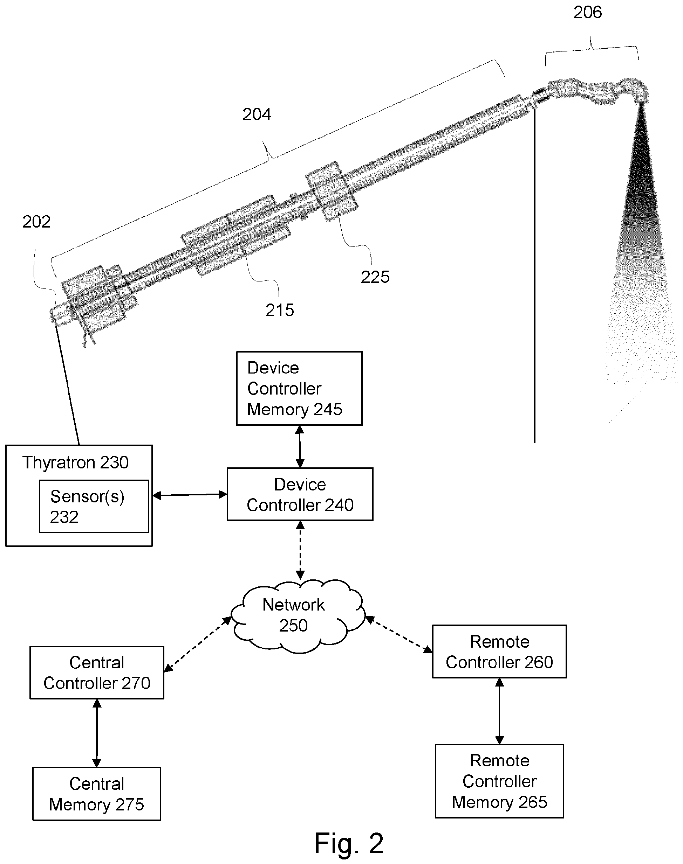

Figure 2 depicts a cross-section through a vacuum tube of a Linac. The specific implementation shown is only one example of a Linac that may form part of a radiotherapy device. A vacuum tube is comprised of an electron gun 202, a waveguide 204, and a flight tube 206. The electron gun 202 is configured to inject electrons into the waveguide 204. In this example, the electron beam may be focused by a first arrangement of focusing magnets 210 and a second arrangement of focusing magnets 215. The beam is 'steered', i.e. directed, by a first arrangement of steering magnets 220 and a second arrangement of steering magnets 225.

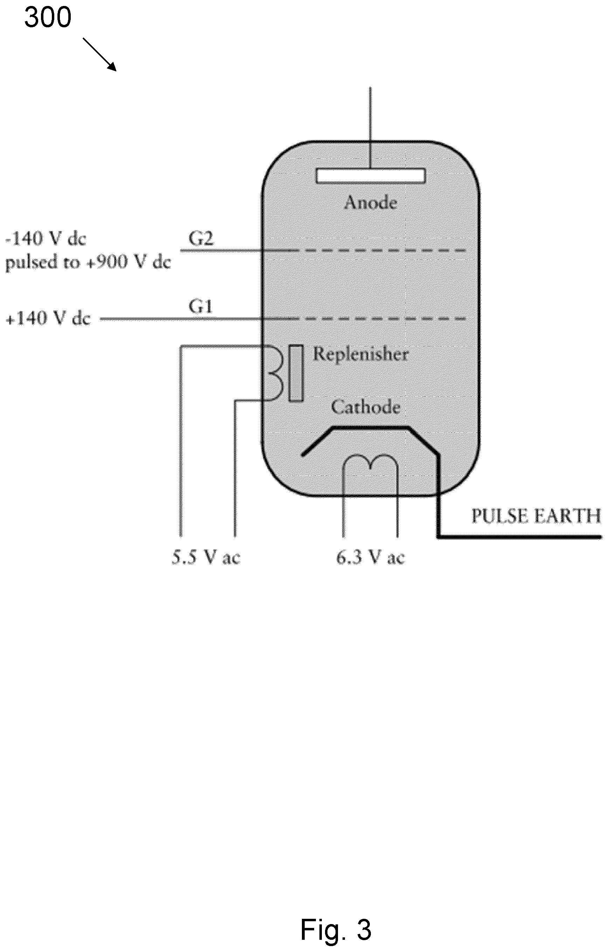

The Linac also comprises a thyratron 230, which in turn comprises one or more sensors 232. In a specific implementation which is described below by reference to figure 3, the one or more sensors are voltage sensors, each suitable for detecting, and configured to detect, voltages associated with the thyratron. The one or more sensors 232 are communicatively coupled with a device controller 240, and data relating to the thyratron voltages is communicated to the device controller 240. The voltage values at the thyratron are measured by the voltage sensors and communicated to the device controller 240 with a particular frequency, for example measurements may be provided every second. The sensors may comprise any number of possible sensors which are suitable to measure voltage.

The Linac device, and in particular the one or more sensors 232, further comprise means with which to communicate with the device controller 240. For example, the one or more sensors 232 may comprise suitable processing circuitry and transmitting antennae. The one or more sensors 232 are electronically and/or communicatively coupled to the device controller 240. The device controller 240 receives signals from the one or more sensors 232 as they are generated, or produced, by the one or more sensors 232. The device controller 240 is electronically and/or communicatively coupled to a device controller memory 245. The device controller 240 and device controller memory 245 may be configured to store signals generated by the one or more sensors 232. The generated signals from the one or more sensors 232 may be referred to as sensor data.

The device controller 240 is communicatively coupled to a central controller 270, for example via a network 250. The device controller 240 is configured to transmit, i.e. send, the sensor data to the central controller 270 to be stored on the central controller memory 275. The central controller memory 275 may comprise a number of different servers as part of a cloud storage solution. The central controller may be communicatively coupled to a plurality of radiotherapy devices via network 250, each of which are configured to transmit signals to the central controller 270 to be stored on central memory 275. The central controller 270 is adapted and configured to process received signals and store them in a database. Processing the signals may comprise, for example, calculating and storing daily averages of particular sensor data.

The signals and sensor data may be stored in the device controller memory 245 and/or may be communicated via the network to the central controller 270. The data may be uploaded to the central controller 270 as it is generated, or may be stored on the device controller memory 245 in order to be uploaded as a batch upload, for example at regular time intervals. Alternatively, the data may be continuously gathered by the device controller 240, for example the sensor signals may be sampled every 4 seconds, optionally while the device is not delivering radiation, and data is uploaded if the data shows a particular variance from the previously uploaded data point. In a specific implementation, the data points are uploaded when there is a change of +/-0.04, and the device controller looks for a new data item once every 4 seconds, optionally while the device is not delivering radiation, and once every second while the Linac is delivering radiation.

The device controller 240 and central controller 270 are both also communicatively coupled to a remote controller 240. The remote controller 260 may access the central database, which stores information and data regarding a plurality of radiotherapy devices, through the database 250 and by using a suitable software platform. The remote controller 260 may also access the device controller 240 to obtain real-time information regarding a particular radiotherapy machine.

The device, central and remote controllers may each be described as a processor, computer, or computing device. The controllers may be connected (e.g., networked) to each other and/or to other machines in a Local Area Network (LAN), an intranet, an extranet, or the Internet. The controllers may each operate in the capacity of a server or a client machine in a client-server network environment, or as a peer machine in a peer-to-peer (or distributed) network environment. The controllers may each be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, respective controllers are illustrated, the term "controller" shall also be taken to include any collection of machines (e.g., computers) that individually or jointly execute a set (or multiple sets) of instructions to perform any one or more of the methodologies discussed herein.

The approaches of the present disclosure may be embodied on one or more of the device controller memory and the remote controller memory, or any other computer-readable medium. The medium may be a non-transitory computer-readable medium. The computer-readable medium carries computer-readable instructions arranged for execution upon a processor so as to make the controller / processor carry out any or all of the methods described herein. The term "computer-readable medium" as used herein refers to any medium that stores data and/or instructions for causing a processor to operate in a specific manner. Such storage medium may comprise non-volatile media and/or volatile media. Non-volatile media may include, for example, optical or magnetic disks. Volatile media may include dynamic memory. Exemplary forms of storage medium include, a floppy disk, a flexible disk, a hard disk, a solid state drive, a magnetic tape, or any other magnetic data storage medium, a CD-ROM, any other optical data storage medium, any physical medium with one or more patterns of holes, a RAM, a PROM, an EPROM, a FLASH-EPROM, NVRAM, and any other memory chip or cartridge.

Figure 3 depicts a thyratron 300 according to the present disclosure. In general, and with reference to figure 2, the radiotherapy device 100 comprises the thyratron 300. A thyratron is used as a high-voltage switch or control element to control, and trigger, the therapeutic beam of radiation. In other words, the thyratron is a high-voltage switch configured to trigger the generation and release of the high-energy electron beam.

The thyratron 300 comprises a cathode and an anode, and a first grid (G1). It further comprises a second grid (G2) and a replenisher. These features of a thyratron, as well as the general function of the thyratron, will be well-known to the skilled person and a detailed discussion is not required herein. However, for the purpose of assisting understanding, a brief explanation of each feature is given below.

The thyratron 300 may comprise, for example, a hydrogen filled tetrode, with a ceramic envelope. The hydrogen gas acts as the switch medium. The hydrogen gas is ionised to make a conductive plasma. The temperature of the cathode is then increased to free the electrons. A first grid (G1) is positive to attract the electrons, which ionises the hydrogen. The grid 1 voltage G1 decreases when there is plasma in the tube, and current flows between the cathode and grid 1. In the off condition, the thyratron has a high voltage. In the on condition, the thyratron initially has a high peak current. Then, by ion recombination, the thyratron quickly goes to the "off" condition. This gives rise to a high fire repetition rate.

The device 100 comprises a high voltage power supply in order to accelerate the electrons in the waveguide 104. This high voltage power supply provides the necessary electric potential to accelerate the electrons to the desired energy level.

In an example, a trigger signal causes a small current to flow through the thyratron. This current causes ionization within the gas-filled tube of the thyratron. The gas inside the thyratron, typically a mixture of gases like hydrogen, helium, mercury or xenon, is initially non-conductive. As ionization progresses, a conductive plasma channel forms inside the thyratron. Conduction increases due to ionization of these gases. This plasma channel allows high voltage to pass through and trigger the switch to the on state. The arc discharge created across the thyratron establishes a low-impedance path for electrons being emitted by the cathode. The temperature of the cathode can then be increased in order to increase the number of electrons being emitted by the cathode, which then flow through the now-conductive thyratron. Alternatively (or additionally), the high-voltage supply can charge a capacitor bank, which is able to discharge energy through the thyratron when the trigger signal is received at the thyratron. This sudden release of energy drives the electron beam acceleration process in the waveguide 104.

The replenisher is a component that serves to maintain the proper gas pressure and composition within the thyratron tube. The replenisher is configured to compensate for any gas loss or degradation that may occur during the operation of the thyratron. Over time, due to factors like gas diffusion or reactions with the tube materials, there may be a gradual depletion or alteration of the gas composition within the thyratron 300. This can affect the tube's performance and reliability. The replenisher is configured to counteract this gas loss by periodically introducing a controlled flow of fresh gas into the thyratron. The proper gas pressure is thereby maintained.

The replenisher may be part of a replenisher system comprising a gas supply, flow control mechanisms, and a pressure regulation system. The gas pressure within the thyratron is monitored, and the replenisher is configured to inject the appropriate amount of fresh gas as needed to maintain the desired operating conditions.

The grids are components that control the flow of electrons through the thyratron 300. The grids modulate and regulate the current passing between the cathode and anode of the thyratron.

The grids may take the form, for example, of a wire mesh positioned between the cathode and anode. The grids are made up of a conductive material that can be negatively biased with respect to the cathode. One or more of the grids may control the ionization and conduction process within the thyratron. By applying a negative bias to the grid, the grid repels the electrons emitted by the cathode. This biasing reduces the number of electrons reaching the anode, preventing premature ionization of the gas in the tube. The grids thereby help maintain the thyratron in its off state until a trigger signal is applied.

When a trigger signal is applied to the thyratron, the grid bias(es) are reduced or removed. This allows the electrons emitted by the cathode to pass through the grid and reach the anode, initiating the conduction of current. Each grid regulates the current flow in the thyratron by adjusting a bias voltage, thereby controlling the number of electrons that can pass through the grid.

The grids also play a role in determining the switching speed of the thyratron. By controlling the grid bias, the time taken to transition from the off state to the on state can be adjusted.

Optionally, the grids can be used for pulse shaping. By applying specific voltage profiles to the grids during conduction, the current waveform can be manipulated. This feature enables pulse characteristics to be tailored and adjusted.

In an example, the first grid voltage is controlled such that ionisation occurs in the tube, and a current passes from the cathode to the first grid. A trigger signal takes the form of pulses received at the second grid. The second grid voltage may thereby be used to control the on or off condition of the thyratron, by either negatively biasing the second grid to repel electrons and thereby keep the thyratron in the 'off' condition, or introducing a large positive voltage to the second grid, thereby accelerating electrons toward the second grid and anode, thereby causing high current to pass through the thyratron as it moves to the "on" condition".

For the thyratron to function optimally, the voltages at each of the cathode, replenisher, the first grid (G1), and the second grid (G2), need to be within certain operating parameters. In an example, operational ranges for each of the parameters may be as follows (Table 1):

| **Parameter** | **Minimum** | **Maximum** |
|---|---|---|
| Replenisher voltage | 5.0V AC | 6.5V AC |
| Cathode heater voltage | 6.0V AC | 6.6V AC |
| Grid 1 voltage | 16.5V DC | 22.0 V DC |
| Grid 2 bias voltages (no pulses) | -120V DC | -160V DC |

These values are examples taken from a specific implementation, and the operational ranges will be different for different implementations. In this example, a cathode heater voltage of 6.3 V AC increases the temperature of the cathode sufficiently to release the electrons. Grid G1 receives a +140 V DC supply which operates the hydrogen ionization. As the hydrogen ionization occurs, the voltage measured on grid 1 decreases to a level of between 12 V and 22 V.

When the pulse forming network (PFN) charges, there is a high voltage between the anode and the cathode. Grid G2 is therefore held negative (-140 V) to isolate the plasma from the high PFN voltage on the anode. This prevents current flow between the cathode and the anode. Grid G2 receives a large positive pulse of +900 V DC for 3 ps, which causes the plasma to expand in the envelope. This causes the thyratron to operate momentarily. When the PFN is fully discharged, the voltage between the anode and the cathode is negligible. The operation of the thyratron stops and is switched off, to prepare for the next charge cycle.

The hydrogen gas is kept as titanium hydride in metal containers in the valve, and is filled by heating the containers. A suitable input to the replenisher may be a 5.5 V AC power supply, which is accurately set to release the correct gas pressure for the operation of the device. As the thyratron ages, the gas pressure decreases and causes the G1 voltage to increase. The gas pressure can be increased if the replenisher voltage is increased.

In usual operation, the hydrogen in the valve envelope gradually diffuses to air, therefore the life of a thyratron usually ends because of the depletion of hydrogen. However, if the replenisher voltage is too high, the hydrogen pressure is too high also and the device may spontaneously conduct (known as fire through). If the replenisher voltage is too low, damage to the anode may result, which also shortens the life of the valve. It is therefore essential that the thyratron is set to operate under the correct conditions.

To date, it has not been thought possible to accurately determine when a thyratron is approaching the end of its operational life. However, the present inventors have realised that it is possible to identify thyratrons which are approaching the end of their operational life by monitoring a "start-up time" or "warm up time" associated with a thyratron. The warm-up time is the time taken for each of the voltages in the above table, e.g. the first grid voltage G1, the second grid voltage G2, the replenisher voltage, and the cathode voltage to reach their acceptable operational ranges, measured from a turn-on-time of the thyratron. At a high-level, the longer a thyratron takes to 'warm up', the more likely it is to break down in the near future. In some instances, to apply a threshold to the warm up time would result in premature action. The present inventors have additionally realised that a rapidly increasing rate of charge in the warm-up time, in combination with a high overall warm-up time, is a highly accurate indicator of whether a thyratron is about to fail.

According to implementations of the present disclosure, the overall warm-up time for a thyratron comprised within a radiotherapy device is monitored. In particular, each time the radiotherapy device and/or thyratron begins the process of 'warming up' ready for use, the time taken for every voltage in Table 1 to reach its respective operational range is recorded. The thyratrons can then be 'labelled', or bucketed, according to its warm-up time. Example labels may be as follows:
Label 1: Thyratrons with a warm-up time exceeding a first time threshold (e.g. a 300 second start up time).
Label 2: Thyratrons with a warm-up time exceeding a second time threshold (e.g. 350 second start up time).
Label 3: Thyratrons with a warm-up time exceeding a third time threshold (e.g. a 400 second start up time).
Label 4: Thyratrons with a warm-up time exceeding a fourth threshold time (e.g. a 450+ second start up time).

In addition to monitoring a warm-up time associated with the thyratron, the rate of change in warm-up time is also calculated and monitored. This may be the change between
successive 'start-ups' of the thyratron, which in a hospital setting typically occur each day. A different rate of change threshold may be applied to the thyratron, depending on its label. In some implementations, the rate of change may be a rolling rate of change calculated over a given time period. For example, it may be calculated over a period of three days, seven days or thirty days. Advantageously, calculating the rolling rate of change over a longer period accounts for normal fluctuations in the start-up time of a thyratron. This approach has been found to be highly accurate not only in identifying thyratrons which are about to fail, but also avoiding false positives. In other words, by applying both an overall threshold, but also a specific and tailored rate of charge threshold according to the overall warm-up time associated with the thyratron, it is possible to identify only those thyratrons which are about to fail.

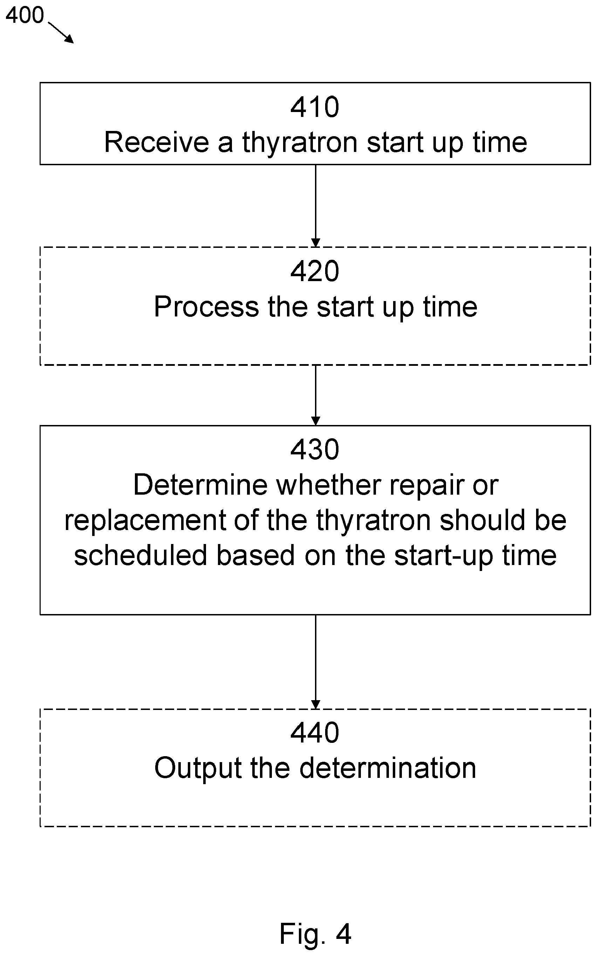

Reference is now made to the method depicted in the flowchart of figure 4. At step 410, a thyratron start-up time is received. The terms "start-up time" and "warm-up time" may be used interchangeably. The start-up time is derived from signals received from one or more sensors 232.

In some implementations, the one or more sensors are voltage sensors, each suitable for detecting, and configured to detect, voltages associated with the thyratron. As set out above, the voltages may be one or more of: the replenisher voltage, a cathode heater voltage, a first grid voltage, and / or a second grid voltage.

Optionally, the signal indicative of the start-up time is based on the value of at least one of the first voltage, the cathode heater voltage, the second voltage, and / or the replenisher voltage. For example, the time taken for the cathode heater voltage to be within acceptable operational parameters may be measured to determine the start-up time.

In preferred implementations, the signals are based on each of the first voltage, the second voltage, the replenisher voltage and the cathode heater voltage each being within a threshold range of values. Each threshold range may be defined by an upper range value and a lower range value which may be different for each respective voltage. The start-up time is measured from the when the thyratron is switched on to the time when each of the first voltage, the second voltage, the replenisher voltage and the cathode heater voltage are within their respective threshold ranges.

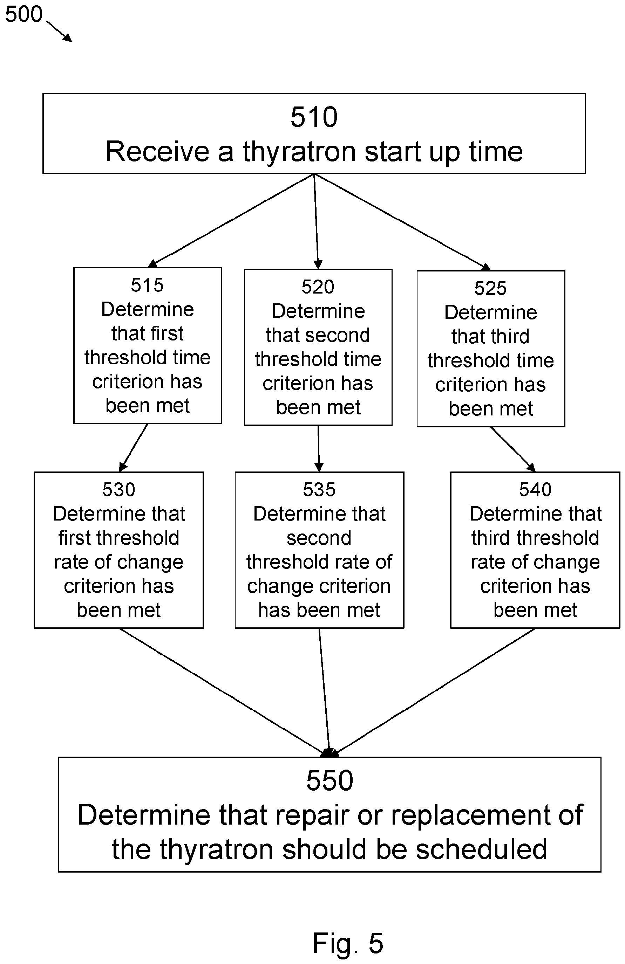

At step 420, the start-up time value may be processed. This is an optional step. The processing of the start-up time is described in more detail in relation to figure 5. In a preferred embodiment, the processing of the start-up time comprises determining whether the start-up time meets at least one threshold time criterion, and determining whether the rate of change of the start-up time has met a threshold rate of change criterion.

At step 430, based on the processing of the start-up time, it is determined whether repair or replacement of the thyratron should be scheduled. In an example, it is determined that repair or replacement should be scheduled if the determinations at step 420 described above are positive, i.e. if the start-up time meets the at least one threshold criterion and the start-up time has changed by at least the threshold amount in a particular time period. For example, the start-up time may exceed 350 seconds and the rate of change over a 30 day rolling period has a linear coefficient greater than 6. A positive determination may be referred to herein as a "hot" label on the thyratron.

In some implementations, the processing may be based on the start-up time meeting a threshold criterion. For example, if the start-up time exceeds 350 seconds, then there is a positive determination that repair or replacement of the thyratron should be scheduled.

By way of a specific example of steps 410, 420, and 430, signals relating to the start-up time of the thyratron are received at the device controller 240 from the sensor 232, and are communicated to the central controller 270 to be stored in the central memory 275. The central controller 270 receives a start-up time for the thyratron every time it is powered on. The rate of change of the start-up time can be calculated or determined based on the received values. In particular, it may be determined what the coefficient of the linear fitting of the start-up time is, and whether it exceeds particular values. Based on the processing of the start-up time data, it is determined whether repair or replacement of the thyratron should be scheduled.

At step 440, the determination at step 430 of whether the repair or replacement should be scheduled is outputted. This step is optional. Outputting the determination may comprise displaying an indication of the determination on a display screen. In response to this indication, the repair or replacement of the thyratron can be scheduled at the next available service point, or at a convenient time for the machine owner, for example during planned machine down time.

Outputting the determination may also comprise issuing an alert or notification detailing the determination to a field service engineer. The alert may comprise information regarding how to test the thyratron for faults, and may also comprise information detailing how to replace the thyratron, or how to repair the thyratron (for example, by re-tapping the replenisher). Outputting the determination may also comprise issuing a communication, alert, or otherwise contacting the owner or operator of the radiotherapy device in order to inform them of the issue and to schedule the repair or replacement.

In an example, once the prediction criteria described herein and set out in the flowchart of figure 5 have been met, a report is generated and an alert is raised. This alert is then sent to the relevant service team and they will plan a replacement, typically within the next 3-6 weeks or align with a planned maintenance activity.

In a preferred embodiment, the device controller 240 receives signals from the current sensor and transmits them to the remote controller 260. The central controller 270 receives the signals and then derives the rate of change of the start-up time, for example by calculating the coefficient of the linear fit of the values over a time period. The coefficient may be stored on the central controller memory 270. Processing of the start-up time then takes place when the remote controller 260 accesses the central memory 275 via the network 250. However, at least some of the steps, and in some examples all of the steps, displayed in figure 4 may be performed on the device controller 240. It will be appreciated that any combination of the device controller 240 central controller 270 and remote controller 260 may be used to perform the disclosed methods.

Reference is now made to the flowchart of figure 5. The flowchart shows the steps 410, 420, and 430 in greater detail, and in particular shows the processing of the start-up time in greater detail. At step 510, a thyratron start-up time is received.

At step 515, it is determined that the received or derived start-up time meets a first threshold time criterion. This may comprise determining that the start-up time for a particular instance of powering up the thyratron is above a first threshold time value. If so, the criterion is met. In a particular example, it is determined whether the start-up time is more than 300 seconds. As an example, the thyratron may be assigned a label 1 if the time exceeds a 300 second start-up time.

At step 540, it is determined that the derived rate of change of the start-up time meets a first threshold rate of change criterion. This may comprise determining that the coefficient of the linear fit of the start-up time values over a time period is above a threshold coefficient value. If so, the criterion is met. In a particular example, after determining that the start-up time is more than 350 seconds, it is determined whether the coefficient is greater than 6.

If both of these thresholds are met at steps 515 and 530, then it will be determined that repair or replacement of the thyratron should be scheduled at step 550. Advantageously, a rapidly increasing rate of charge in the warm-up time, in combination with a high overall warm-up time, is a highly accurate indicator of whether a thyratron is about to fail.

At step 520, it is determined that the received or derived start-up time meets a second threshold time criterion. At step 535, it is determined that the derived rate of change of the start-up time meets a second threshold rate of change criterion. In a particular example, it is determined that the start-up time is more than 400 seconds, and the coefficient of the rate of change is greater than 4.

At step 525, it is determined that the received or derived start-up time meets a third threshold time criterion. At step 540, it is determined that the derived rate of change of the start-up time meets a fourth threshold rate of change criterion. In a particular example, it is determined that the start-up time is more than 450 seconds, and the coefficient of the rate of change is greater than 2.

The first threshold time criterion may be less than the second threshold time criterion, and the second threshold time criterion may be less than the third threshold time criterion. The first threshold rate of change criterion may be greater than the second threshold rate of change criterion, and the second threshold rate of change criterion may be greater than the third threshold rate of change criterion. In other words, if a lower threshold time criterion is met, then the rate of change of the start-up time must be greater for the "hot" label to be assigned to the thyratron and for there to be a determination that repair or replacement of the thyratron should be scheduled at step 550.

In an example, processing may comprise determining whether the start-up time meets a first threshold criterion (for example, a time threshold of 350 second) and determining whether the rate of change of the start-up time meets a first threshold rate of change criterion (for example, a rate of change coefficient of 6). If both of these criteria are met, then it may be determined that repair or replacement of the thyratron should be scheduled.

In a further example, it may be determined that the first threshold rate of change criterion is not met (for example, the rate of change coefficient is less than 6). It may be further determined whether the start-up time meets a second threshold time criterion (for example, a time threshold of 450 seconds). The second threshold time criterion is greater than the first threshold time criterion. It may be determined whether the rate of change of the start-up time meets a second threshold rate of change criterion (for example, a rate of change coefficient of 2). The second threshold rate of change criterion is less than the first threshold rate of change criterion (for example, a rate of change coefficient of 4). If the second threshold time criterion and the second threshold rate of change criterion are met, it may be determined that repair or replacement of the thyratron should be scheduled.

In some implementations, the "hot" label may be assigned to a given thyratron based on additional alarms or alerts. These may indicate other issues with the thyratron that are not linked directly to the start-up time. One example of an alarm is a GTO O/Load. Another example of an alarm is a Thyratron grid Inhibit. The voltage may have become mismatched or be outside their existing ranges. This may indicate that the replenisher voltage of the thyratron needs to be adjusted, or another repair is needed.

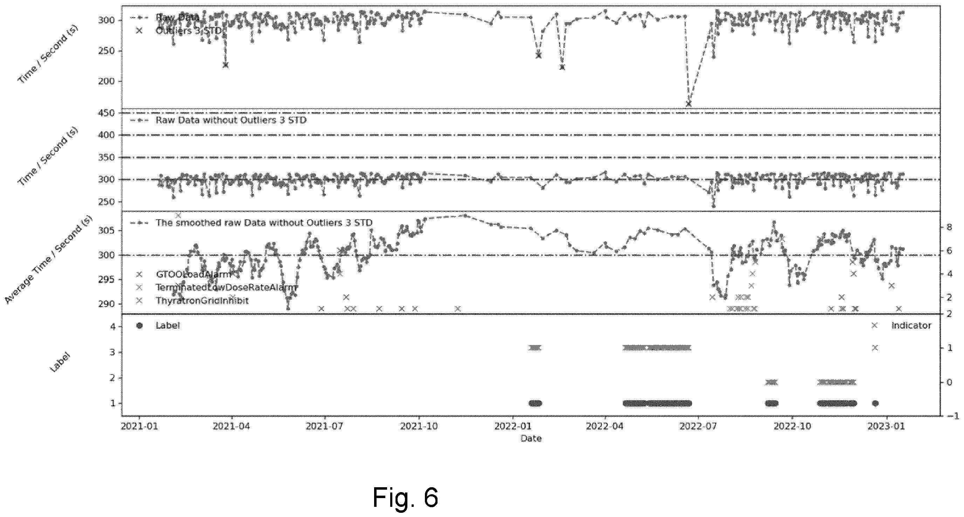

Figure 6 depicts received start-up time data for a thyratron over a time period, for a thyratron assigned label 1. This label may be assigned when the first threshold criterion (for example, 350 seconds) has not been met, and therefore the thyratron will not be assigned to be "hot". However, the data may exceed a different threshold, such as 300 seconds. This may indicate that although a thyratron is not yet "hot" i.e., repair or replacement does not need to be scheduled, it may need to be monitored more closely in future.

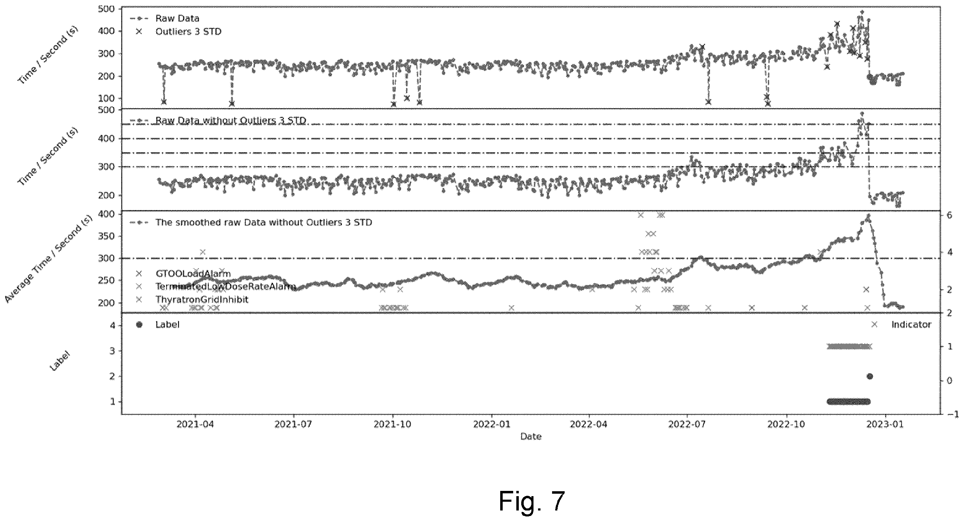

Figure 7 depicts received start-up time data for a thyratron over a time period, for a thyratron assigned label 2. This label may be assigned when the first threshold criterion (for example, 350 seconds) has been reached.

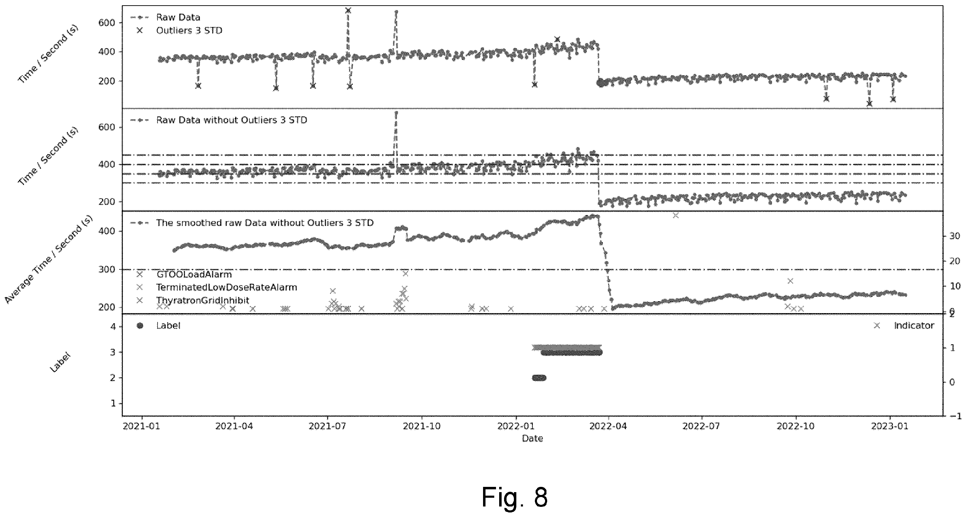

Figure 8 depicts received start-up time data for a thyratron over a time period, for a thyratron assigned label 3. This label may be assigned when the second threshold criterion (for example, 400 seconds) has been reached.

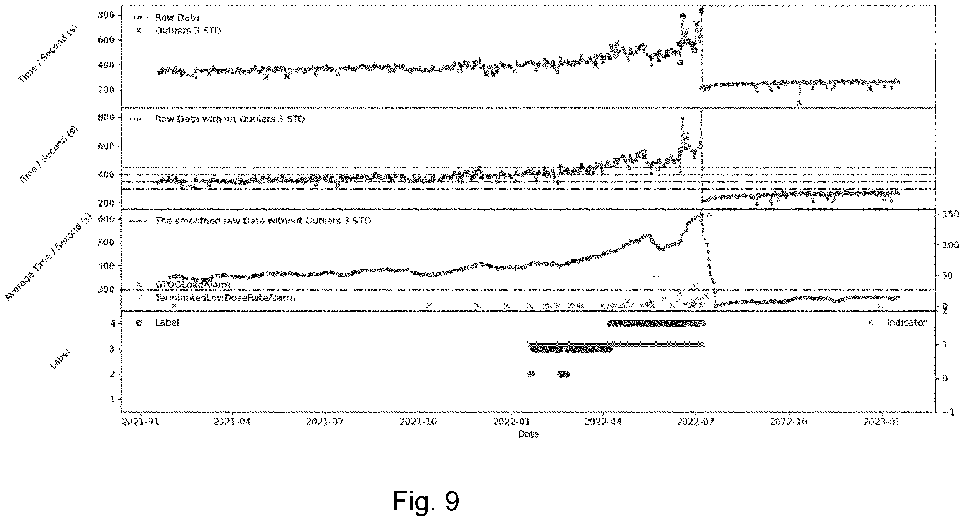

Figure 9 depicts received start-up time data for a thyratron over a time period, for a thyratron assigned label 4. This label may be assigned when the third threshold criterion (for example, 450 seconds) has been reached.

Example threshold values are depicted by dotted and dashed lines in Figures 6 to 9. The first threshold time criterion is shown at 350 seconds for label 2, the second threshold time criterion is shown at 400 seconds for label 3, and the third threshold time criterion is shown at 450 seconds for label 4. A further threshold is shown at 300 seconds for label 1.

Rate of change may be calculated based on data points, depicted in Figures 6 to 9 as solid circles linked by dashed lines. In some implementations, the rate of change may be calculated based on a time averaged start-up time. This start-up time may be an average of signals received from the sensor over an averaging time period.

Optionally, processing the start-up time may comprise excluding outliers. There may be extreme values of start-up time that are measured that are not indicative that a thyratron is about to fail. Processing steps such as excluding outliers, time averaging data points, and calculating a rolling rate of change contribute to a more accurate prediction of the operation of a thyratron.

In the example of Figure 6, a label of 1 is assigned for data that falls between the thresholds of 300 and 350 seconds. In the example of Figure 7, a label of 2 is assigned for data that falls between the thresholds of 350 and 400 seconds. In the example of Figure 8, a label of 3 is assigned for data that falls between the thresholds of 400 and 450. In the example of Figure 9, a label of 4 is assigned for data that is above the threshold of 450. These labels are indicative of a category that the data falls into, although the upper and lower values of the thresholds may change depending on the parameters of the thyratron.

The rate of change criterion applied to the data may be selected based on the label assigned. For example, a label 2 thyratron must be determined to have a rate of change with a coefficient greater than 6 to be determined to be "hot". However, a label 4 thyratron only needs to have a rate of change with a coefficient greater than 2 to be determined to be "hot". Advantageously, using both sets of criterion means that a more accurate determination of how likely a thyratron is to need repair or replacement. The machine downtime, as well as the cost associated with parts and labour are therefore reduced.

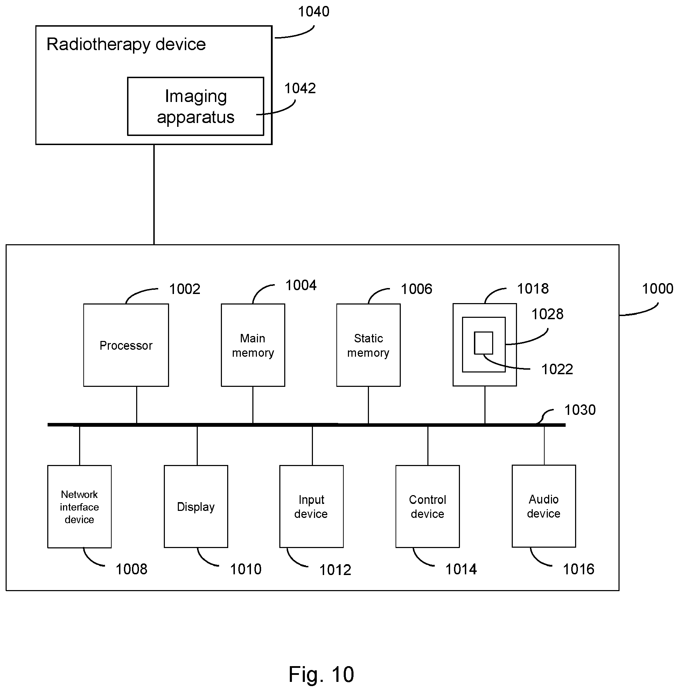

Figure 10 illustrates a block diagram of one implementation of a computing device 1000 within which a set of instructions for causing the computing device and/or the radiotherapy device 1040 to perform any one or more of the methodologies discussed herein, may be executed. The computing device 1000 may form part of a radiotherapy device 1040, apparatus, or system, or may be communicatively coupled to the radiotherapy device 1040. In implementations, the computing device may be connected (e.g., networked) to other machines, including other radiotherapy devices, in a Local Area Network (LAN), an intranet, an extranet, or the Internet. The computing device may operate in the capacity of a server or a client machine in a client-server network environment, or as a peer machine in a peer-to-peer (or distributed) network environment. The computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 1000 includes a processing device 1002, a main memory 1004 (e.g., read-only memory (ROM), flash memory, dynamic random access memory (DRAM) such as synchronous DRAM (SDRAM) or Rambus DRAM (RDRAM), etc.), a static memory 1006 (e.g., flash memory, static random access memory (SRAM), etc.), and a secondary memory (e.g., a data storage device 1018), which communicate with each other via a bus 1030.

Processing device 1002 represents one or more general-purpose processors such as a microprocessor, central processing unit, or the like. More particularly, the processing device 1002 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 1002 may also be one or more special-purpose processing devices such as an application specific integrated circuit (ASIC), a field programmable gate array (FPGA), a digital signal processor (DSP), network processor, or the like. Processing device 1002 is configured to execute the processing logic (instructions 1022) for performing the operations and steps discussed herein.

The device controller 240, central controller 270 and remote controller 260 of Figure 2 may each be described as a processor 1002. The controllers may be connected (e.g., networked) to each other and/or to other machines in a Local Area Network (LAN), an intranet, an extranet, or the Internet. As set out above, signals and sensor data may be stored in the device controller memory 245 and/or may be communicated via the network to the central controller 270. The data may be uploaded to the central controller 270 as it is generated, or may be stored on the device controller memory 245 in order to be uploaded as a batch upload, for example at regular time intervals. The remote controller 260 may access the central database, which stores information and data regarding a plurality of radiotherapy devices, through the database 250 and by using a suitable software platform. The remote controller 260 may also access the device controller 240 to obtain real-time information regarding a particular radiotherapy machine.

The computing device 1000 may further include a network interface device 1008. The computing device 1000 also may include a video display unit 1010 (e.g., a liquid crystal display (LCD) or a cathode ray tube (CRT)), an alphanumeric input device 1012 (e.g., a keyboard or touchscreen), a cursor control device 1014 (e.g., a mouse or touchscreen), and an audio device 1016 (e.g., a speaker). The network interface device 1008 may be located in the treatment room, or in another room such as a nearby room outside the radiotherapy bunker.

The data storage device 1018 may include one or more machine-readable storage media (or more specifically one or more non-transitory computer-readable storage media) 1028 on which is stored one or more sets of instructions 1022 embodying any one or more of the methodologies or functions described herein. The instructions 1022 may also reside, completely or at least partially, within the main memory 1004 and/or within the processing device 1002 during execution thereof by the computer system 1000, the main memory 1004 and the processing device 1002 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as " receiving", "determining", "comparing ", "enabling", "maintaining," "identifying," or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

Embodiments of the present disclosure are set out in the following numbered clauses:
Clause 1. A method of determining whether repair or replacement of thyratron of a radiotherapy device should be scheduled, the radiotherapy device comprising a linear accelerator and being configured to provide therapeutic radiation to a patient, the radiotherapy device comprising:
   a thyratron; and
   at least one sensor configured to provide a signal indicative of a start-up time associated with the thyratron;
   the method comprising:
      receiving the start-up time;
      based on the start-up time, determining whether repair or replacement of the thyratron should be scheduled.
Clause 2. The method of clause 1, further comprising processing the start-up time and based on the processing, determining whether repair or replacement of the thyratron should be scheduled.
Clause 3. The method of clause 2, wherein processing the start-up time comprises: determining whether the start-up time meets at least one threshold time criterion.
Clause 4. The method of clause 2 or 3, wherein processing the start-up time comprises: determining whether a rate of change of the start-up time meets at least one threshold rate of change criterion.
Clause 5. The method of clause 4, wherein processing the start-up time comprises:
   determining whether the start-up time meets a first threshold criterion; and
   determining whether the rate of change of the start-up time meets a first threshold rate of change criterion;
   if the first threshold time criterion and first threshold rate of change criterion are met, determining that repair or replacement of the thyratron should be scheduled.
Clause 6. The method of clause 5, wherein processing the start-up time comprises:
   determining that the first threshold rate of change criterion is not met; and
   determining whether the start-up time meets a second threshold time criterion, wherein the second threshold time criterion is greater than the first threshold time criterion;
   determining whether the rate of change of the start-up time meets a second threshold rate of change criterion, wherein the second threshold rate of change criterion is less than the first threshold rate of change criterion;
   if the second threshold time criterion and the second threshold rate of change criterion are met, determining that repair or replacement of the thyratron should be scheduled.
Clause 7. The method of any preceding clause, wherein the thyratron comprises:
   an anode;
   a cathode heater, wherein a cathode heater voltage is associated with the cathode heater;
   a first grid positioned between the cathode and the anode, wherein a first voltage is associated with the first grid.
Clause 8. The method of any preceding clause, wherein the thyratron comprises a second grid positioned between the cathode and the anode, wherein a second voltage is associated with the second grid.
Clause 9. The method of any preceding clause, wherein the thyratron comprises a replenisher, wherein a replenisher voltage is associated with the replenisher.
Clause 10. The method of any of clauses 7 to 9, wherein the signal indicative of the start-up time is based on the value of at least one of the first voltage, the cathode heater voltage, the second voltage, and / or the replenisher voltage.
Clause 11. The method of clause 10, wherein the signals indicative of the start-up time are based on each of the first voltage, the second voltage, the replenisher voltage and the cathode heater voltage each being within a threshold range of values, each threshold range being defined by an upper range value and a lower range value.
Clause 12. The method of any preceding clause, wherein the start-up time is an average of signals received from the sensor over an averaging time period.
Clause 13. The method of any preceding clause, wherein determining whether the rate of change meets the threshold rate of change criterion comprises calculating a linear coefficient of the rate of change of the start-up time over a rolling time period.
Clause 14. The method of clause 13, wherein the rolling time period is greater than 24 hours, and optionally greater than three days, and optionally greater than seven days, and optionally greater than thirty days.
Clause 15. The method of any preceding clause, further comprising outputting the determination of whether repair or replacement of the thyratron should be scheduled.
Clause 16. The method of clause 11, wherein outputting the determination comprises at least one of displaying an indication of the determination on a display screen; issuing an alert detailing the determination to a field service engineer; and/or automatically issuing a notification to the owner of the radiotherapy device to schedule the repair or replacement
Clause 17. A computer-readable medium comprising computer-executable instructions which, when executed by a processor, cause the processor to perform the method of any preceding clause.
Clause 18. A system comprising a remote controller communicatively coupled to a central controller and a device controller via a network, the central controller configured to receive data from a radiotherapy device, the radiotherapy device comprising a linear accelerator, the radiotherapy device being configured to provide therapeutic radiation to a patient, and the radiotherapy device comprising:
   a thyratron and a sensor configured to provide at least one signal indicative of start-up time of the thyratron;
   wherein the central controller, the remote controller and/or a device controller are configured to:
      receive the start-up time;
      based on the start-up time, determine whether repair or replacement of the thyratron should be scheduled.
Clause 19. The system of clause 18, wherein the central controller, the remote controller and/or a device controller are further configured to: process the start-up time and based on the processing, determine whether repair or replacement of the thyratron should be scheduled.
Clause 20. The system of clause 19, wherein processing the start-up time comprises: determining whether the start-up time meets at least one threshold time criterion.
Clause 21. The system of clause 19 or 20, wherein processing the start-up time comprises: determining whether a rate of change of the start-up time meets at least one threshold rate of change criterion.
Clause 22. The system of clause 21, wherein processing the start-up time comprises:
   determining whether the start-up time meets a first threshold criterion; and
   determining whether the rate of change of the start-up time meets a first threshold rate of change criterion;
   if the first threshold time criterion and first threshold rate of change criterion are met, determining that repair or replacement of the thyratron should be scheduled.
Clause 23. The system of clause 22, wherein processing the start-up time comprises:
   determining that the first threshold rate of change criterion is not met; and
   determining whether the start-up time meets a second threshold time criterion, wherein the second threshold time criterion is greater than the first threshold time criterion;
   determining whether the rate of change of the start-up time meets a second threshold rate of change criterion, wherein the second threshold rate of change criterion is less than the first threshold rate of change criterion;
   if the second threshold time criterion and the second threshold rate of change criterion are met, determining that repair or replacement of the thyratron should be scheduled.
Clause 24. The system of any of clauses 18-23, wherein the thyratron comprises:
   an anode;
   a cathode heater, wherein a cathode heater voltage is associated with the cathode heater;
   a first grid positioned between the cathode and the anode, wherein a first voltage is associated with the first grid.
Clause 25. The system of any of clauses 18-24, wherein the thyratron comprises a second grid positioned between the cathode and the anode, wherein a second voltage is associated with the second grid.
Clause 26. The system of any of clauses 18-25, wherein the thyratron comprises a replenisher, wherein a replenisher voltage is associated with the replenisher.
Clause 27. The system of any of clauses 24 to 26, wherein the signal indicative of the start-up time is based on the value of at least one of the first voltage, the cathode heater voltage, the second voltage, and / or the replenisher voltage.
Clause 28. The system of clause 27, wherein the signals indicative of the start-up time are based on each of the first voltage, the second voltage, the replenisher voltage and the cathode heater voltage each being within a threshold range of values, each threshold range being defined by an upper range value and a lower range value.
Clause 29. The system of any of clauses 18-28, wherein the start-up time is an average of signals received from the sensor over an averaging time period.
Clause 30. The system of any of clauses 18-29, wherein determining whether the rate of change meets the threshold rate of change criterion comprises calculating a linear coefficient of the rate of change of the start-up time over a rolling time period.
Clause 31. The system of clause 30, wherein the rolling time period is greater than 24 hours, and optionally greater than three days, and optionally greater than seven days, and optionally greater than thirty days.
Clause 32. The system of any of clauses 18-31, wherein the central controller, the remote controller and/or the device controller are further configured to output the determination of whether repair or replacement of the thyratron should be scheduled.
Clause 33. The system of clause 32, wherein outputting the determination comprises at least one of displaying an indication of the determination on a display screen; issuing an alert detailing the determination to a field service engineer; and/or automatically issuing a notification to the owner of the radiotherapy device to schedule the repair or replacement.

## Claims

1. A method of determining whether repair or replacement of thyratron of a radiotherapy device should be scheduled, the radiotherapy device comprising a linear accelerator and being configured to provide therapeutic radiation to a patient, the radiotherapy device comprising:
a thyratron; and
at least one sensor configured to provide a signal indicative of a start-up time associated with the thyratron;
the method comprising:
receiving the start-up time;
based on the start-up time, determining whether repair or replacement of the thyratron should be scheduled.

2. The method of claim 1, further comprising processing the start-up time and based on the processing, determining whether repair or replacement of the thyratron should be scheduled.

3. The method of claim 2, wherein processing the start-up time comprises:
determining whether the start-up time meets at least one threshold time criterion.

4. The method of claim 2 or 3, wherein processing the start-up time comprises:
determining whether a rate of change of the start-up time meets at least one threshold rate of change criterion.

5. The method of claim 4, wherein processing the start-up time comprises:
determining whether the start-up time meets a first threshold criterion; and
determining whether the rate of change of the start-up time meets a first threshold rate of change criterion;
if the first threshold time criterion and first threshold rate of change criterion are met,
determining that repair or replacement of the thyratron should be scheduled.

6. The method of claim 5, wherein processing the start-up time comprises:
determining that the first threshold rate of change criterion is not met; and
determining whether the start-up time meets a second threshold time criterion,
wherein the second threshold time criterion is greater than the first threshold time criterion;
determining whether the rate of change of the start-up time meets a second threshold rate of change criterion, wherein the second threshold rate of change criterion is less than the first threshold rate of change criterion;
if the second threshold time criterion and the second threshold rate of change criterion are met, determining that repair or replacement of the thyratron should be scheduled.

7. The method of any preceding claim, wherein the thyratron comprises:
an anode;
a cathode heater, wherein a cathode heater voltage is associated with the cathode heater;
a first grid positioned between the cathode and the anode, wherein a first voltage is associated with the first grid.

8. The method of any preceding claim, wherein the thyratron comprises a second grid positioned between the cathode and the anode, wherein a second voltage is associated with the second grid.

9. The method of any preceding claim, wherein the thyratron comprises a replenisher, wherein a replenisher voltage is associated with the replenisher.

10. The method of any of claims 7 to 9, wherein the signal indicative of the start-up time is based on the value of at least one of the first voltage, the cathode heater voltage, the second voltage, and / or the replenisher voltage.

11. The method of claim 10, wherein the signals indicative of the start-up time are based on each of the first voltage, the second voltage, the replenisher voltage and the cathode heater voltage each being within a threshold range of values, each threshold range being defined by an upper range value and a lower range value.

12. The method of any preceding claim, wherein the start-up time is an average of signals received from the sensor over an averaging time period.

13. The method of any preceding claim, wherein determining whether the rate of change meets the threshold rate of change criterion comprises calculating a linear coefficient of the rate of change of the start-up time over a rolling time period.

14. A computer-readable medium comprising computer-executable instructions which, when executed by a processor, cause the processor to perform the method of any preceding claim.

15. A system comprising a remote controller communicatively coupled to a central controller and a device controller via a network, the central controller configured to receive data from a radiotherapy device, the radiotherapy device comprising a linear accelerator, the radiotherapy device being configured to provide therapeutic radiation to a patient, and the radiotherapy device comprising:
a thyratron and a sensor configured to provide at least one signal indicative of start-up time of the thyratron;
wherein the central controller, the remote controller and/or a device controller are configured to perform the method steps of any of claims 1 to 13.
